# EUROPEAN PATENT APPLICATION

(11) **EP 3 499 508 A1**
(43) Date of publication of application: **19.06.2019**
(21) Application number: 17207389.2
(22) Date of filing: 14.12.2017
(51) Int. Cl.: G16H 20/70

(54) **COMPUTER-IMPLEMENTED METHOD AND APPARATUS FOR GENERATING INFORMATION**

(71) Applicant: Koninklijke Philips N.V., 5656 AE Eindhoven (NL)
(72) Inventor: FULTON, Paul Michael, 5656 AE Eindhoven (NL); TEN KATE, Warner Rudolph Theophile, 5656 AE Eindhoven (NL); GEURTS, Lucas Jacobus Franciscus, 5656 AE Eindhoven (NL); DU, Jia, 5656 AE Eindhoven (NL); HEESEMANS, Michael, 5656 AE Eindhoven (NL)
(74) Representative: de Haan, Poul Erik

(57) **Abstract**

According to an aspect, there is provided a computer-implemented method to generate information for a first user, the method comprising storing values for one or more parameters for each of a plurality of possible conversation topics for a second user in a first database; obtaining measurements of recent activities and/or behaviours of the second user; using information from a knowledge base that provides associations between conversation topics and types of activities and/or behaviours to identify one or more of the possible conversation topics that relate to the recent activities and/or behaviours; updating the stored values for one or more parameters for the identified one or more possible conversation topics based on the measurements of recent activities and/or behaviours; and when information is to be generated for the first user: ranking each of the possible conversation topics according to a predetermined function and the stored values for each possible conversation topic; selecting one or more of the possible conversation topics according to the ranking; and outputting the selected conversation topics to be presented to the first user.

## Description

### FIELD OF THE INVENTION

This disclosure relates to a computer-implemented method and apparatus for generating information for a first user, and in particular information relating to one or more conversation topics for use with a second user.

### BACKGROUND OF THE INVENTION

Visiting or caring for someone with dementia or other conditions involving significant cognitive problems is associated with many difficulties. One particular issue is maintaining a comfortable and productive conversation, particularly where feedback from the person with dementia may be limited or affected by their condition. Maintaining social contact is just as important for people with such conditions as for anyone else to ensure that the person remains engaged with their life and surroundings, since lack of social contact may affect health and wellbeing, for example leading to depression. Increasing patient-caregiver engagement helps the (formal or informal) caregiver to provide better care with more positive outcomes. It also helps to build up trust and rapport in the communication with the person with cognitive problem(s). However, a visitor is likely to find maintaining a comfortable and productive conversation difficult and uncomfortable with a person that they do not know well or with a person that has limited conversation abilities due to their condition. Other than asking an expert for advice, solutions to help both parties in such situations are limited.

There is therefore a need for methods and apparatus that can provide or suggest suitable conversation topics to a user to enable them to improve their conversations with another user, thereby improving the social contact with the other user.

### SUMMARY OF THE INVENTION

According to a first specific aspect, there is provided a computer-implemented method to generate information for a first user, the method comprising storing, via a processing unit (e.g. a processor, a microprocessor), values for one or more parameters for each of a plurality of possible conversation topics for a second user in a first database; obtaining, via the processing unit, measurements of recent activities and/or behaviours of the second user; using, via the processing unit, information from a knowledge base that provides associations between conversation topics and types of activities and/or behaviours to identifyone or more of the possible conversation topics that relate to the recent activities and/or behaviours; updating, via the processing unit, the stored values for one or more parameters for the identified one or more possible conversation topics based on the measurements of recent activities and/or behaviours; and when information is to be generated for the first user: ranking, via the processing unit, each of the possible conversation topics according to a predetermined function and the stored values for each possible conversation topic; selecting, via the processing unit, one or more of the possible conversation topics according to the ranking; and outputting the selected conversation topics to be presented to the first user. Thus this aspect overcomes some of the difficulties in talking to people with impaired memory, or in talking to unfamiliar people, making conversation easier and more comfortable for all participants. More specifically, this aspect helps with the question "What should I talk about next?" for a person (i.e. the first person) that is getting limited feedback from the other person (i.e. the second person).

In some embodiments, the one or more parameters stored in the first database for each of the plurality of possible conversation topics for the second user relate to one or more of: an importance of the conversation topic to the first user and/or the second user; how recently the conversation topic was used for the second user; a duration of a previous conversation with the second user on the conversation topic; and how frequently the conversation topic is used for the second user.

In some embodiments, the step of ranking each of the possible conversation topics comprises using the predetermined function and the stored values for each conversation topic to determine a ranking value for each conversation topic; and the step of selecting one or more of the possible conversation topics according to the ranking comprises selecting a predetermined number of the possible conversation topics having the highest determined ranking values.

In alternative embodiments, the step of ranking each of the possible conversation topics comprises using the predetermined function and the stored values for each conversation topic to determine a ranking value for each conversation topic; and the step of selecting one or more of the possible conversation topics according to the ranking comprises comparing the determined ranking value to a threshold value. In these embodiments, the step of selecting comprises selecting all or a subset of the possible conversation topics for which the determined ranking value exceeds the threshold value.

In some embodiments, the predetermined function comprises determining, for each possible conversation topic for which an associated recent activity and/or behaviour has been measured, a normality value that provides a measure of how normal or abnormal the associated recent activity and/or behaviour is for the second user.

In some embodiments, the step of ranking each of the possible conversation topics comprises, for each conversation topic in the first database, searching an external information source for information relevant to the conversation topic; and the predetermined function comprises determining a weighting value for each conversation topic based on information relevant to the conversation topic that is found in the search of the external information source.

In some embodiments, the knowledge base stores one or more ontologies that provide associations between conversation topics and types of activities and/or behaviours.

In some embodiments, the step of providing the first database further comprises storing, for one or more of the plurality of possible conversation topics, information on recent and previous activities and/or behaviours of the second user relating to the conversation topic.

In some embodiments, the method further comprises evaluating, via the processing unit, the obtained measurements of the recent activities and/or behaviours of the second user to determine whether any of the recent activities and/or behaviours are abnormal for the second user; and storing, via the processing unit, for one or more of the plurality of possible conversation topics, information on determined abnormal recent activities and/or behaviours of the second user relating to the conversation topic. This information on abnormal recent activities and/or behaviours can be used to influence the ranking value for associated conversation topics, thereby increasing the likelihood that the first user will be presented with conversation topics associated with the abnormal activities and/or behaviour to enable the first user to find out more about those activities and/or behaviours.

In some embodiments, the method further comprises providing, via the processing unit, a second database that stores the plurality of possible conversation topics for the second user. In these embodiments, the method further comprises searching an external information source for external information relevant to the second user; updating the second database with one or more new conversation topics relating to the external information; and updating the first database to store values for the one or more parameters for each of the one or more new conversation topics. In this way, the possible conversation topics can be updated based on current events, e.g. news or weather, and/or the possible conversation topics can be enhanced using information obtained from the Internet or other sources.

In some embodiments, the method further comprises obtaining, via the processing unit, user profile information relating to the second user, the user profile information indicating importance values for one or more of the plurality of possible conversation topics for the second user; and updating, via the processing unit, the stored values for the one or more parameters for the one or more of the plurality of possible conversation topics for the second user based on the user profile information. In this way, the ranking value, and thus the likelihood that the conversation topic will be presented to the first user, can take into account preferences of the second user.

In some embodiments, the method further comprises, after presenting the selected conversation topics to the first user, updating, via the processing unit, the stored values for the one or more parameters for the selected conversation topics. In this way, the chances of the method selecting the same conversation topics each time will be reduced.

In some embodiments, the method further comprises receiving, via the processing unit, feedback from the first user and/or the second user on the selected conversation topics; and updating, via the processing unit, the stored values for the one or more parameters for the selected conversation topics based on the received feedback. In this way, the chances of selecting conversation topics that have previously led to a positive conversation can be increased, while the chances of selecting conversation topics that have previously failed to lead to a positive conversation can be reduced.

According to a second aspect, there is provided a computer program product comprising a computer readable medium having computer readable code embodied therein, the computer readable code being configured such that, on execution by a suitable computer or processor, the computer or processor is caused to perform the method of the first aspect or any embodiment thereof.

According to a third aspect, there is provided an apparatus for generating information for a first user, the apparatus comprising a processing unit that is configured to interface with a first database that stores values for one or more parameters for each of a plurality of possible conversation topics for a second user; obtain measurements of recent activities and/or behaviours of the second user; use information from a knowledge base that provides associations between conversation topics and types of activities and/or behaviours to identify one or more of the possible conversation topics that relate to the recent activities and/or behaviours; update the stored values for one or more parameters for the identified one or more possible conversation topics based on the measurements of recent activities and/or behaviours; rank each of the possible conversation topics in the first database according to a predetermined function and the stored values for each possible conversation topic when information is to be generated for the first user; select one or more of the possible conversation topics according to the ranking; and provide a signal to a user interface to present the selected conversation topics to the first user. Thus this aspect overcomes some of the difficulties in talking to people with impaired memory, or in talking to unfamiliar people, making conversation easier and more comfortable for all participants. More specifically, this aspect helps with the question "What should I talk about next?" for a person that is getting limited feedback from the other person.

In some embodiments, the one or more parameters stored in the first database for each of the plurality of possible conversation topics for the second user relate to one or more of: an importance of the conversation topic to the first user and/or the second user; how recently the conversation topic was used for the second user; a duration of a previous conversation with the second user on the conversation topic; and how frequently the conversation topic is used for the second user.

In some embodiments, the processing unit is configured to rank each of the possible conversation topics by using the predetermined function and the stored values for each conversation topic to determine a ranking value for each conversation topic; and the processing unit is configured to select one or more of the possible conversation topics according to the ranking by selecting a predetermined number of the possible conversation topics having the highest determined ranking values.

In alternative embodiments, the processing unit is configured to rank each of the possible conversation topics by using the predetermined function and the stored values for each conversation topic to determine a ranking value for each conversation topic; and the processing unit is configured to select one or more of the possible conversation topics according to the ranking by comparing the determined ranking value to a threshold value. In these embodiments, the processing unit is configured to select all or a subset of the possible conversation topics for which the determined ranking value exceeds the threshold value.

In some embodiments, the predetermined function determines, for each possible conversation topic for which an associated recent activity and/or behaviour has been measured, a normality value that provides a measure of how normal or abnormal the associated recent activity and/or behaviour is for the second user.

In some embodiments, the processing unit is configured to rank each of the possible conversation topics by, for each conversation topic in the first database, searching an external information source for information relevant to the conversation topic; and the predetermined function comprises determining a weighting value for each conversation topic based on information relevant to the conversation topic that is found in the search of the external information source.

In some embodiments, the knowledge base stores one or more ontologies that provide associations between conversation topics and types of activities and/or behaviours.

In some embodiments, the first database further stores, for one or more of the plurality of possible conversation topics, information on recent and previous activities and/or behaviours of the second user relating to the conversation topic.

In some embodiments, the processing unit is configured to evaluate the obtained measurements of the recent activities and/or behaviours of the second user to determine whether any of the recent activities and/or behaviours are abnormal for the second user; and store, for one or more of the plurality of possible conversation topics, information on determined abnormal recent activities and/or behaviours of the second user relating to the conversation topic. This information on abnormal recent activities and/or behaviours can be used to influence the ranking value for associated conversation topics, thereby increasing the likelihood that the first user will be presented with conversation topics associated with the abnormal activities and/or behaviour to enable the first user to find out more about those activities and/or behaviours.

In some embodiments, the processing unit is further configured to interface with a second database that stores the plurality of possible conversation topics for the second user. In these embodiments, the processing unit is configured to initiate a search of an external information source for external information relevant to the second user; update the second database with one or more new conversation topics relating to the external information; and update the first database to store values for the one or more parameters for each of the one or more new conversation topics. In this way, the possible conversation topics can be updated based on current events, e.g. news or weather, and/or the possible conversation topics can be enhanced using information obtained from the Internet or other sources.

In some embodiments, the processing unit is configured to receive user profile information relating to the second user, the user profile information indicating importance values for one or more of the plurality of possible conversation topics for the second user; and update the stored values for the one or more parameters for the one or more of the plurality of possible conversation topics for the second user based on the user profile information. In this way, the ranking value, and thus the likelihood that the conversation topic will be presented to the first user, can take into account preferences of the second user.

In some embodiments, the processing unit is further configured to, after presenting the selected conversation topics to the first user, update the stored values for the one or more parameters for the selected conversation topics. In this way, the chances of the apparatus selecting the same conversation topics each time will be reduced.

In some embodiments, the processing unit is further configured to receive feedback from the first user and/or the second user on the selected conversation topics; and update the stored values for the one or more parameters for the selected conversation topics based on the received feedback. In this way, the chances of selecting conversation topics that have previously led to a positive conversation can be increased, while the chances of selecting conversation topics that have previously failed to lead to a positive conversation can be reduced.

According to a fourth aspect, there is provided a system comprising an apparatus according to the third aspect or any embodiment thereof; and one or more of: the first database that is configured to store values for one or more parameters for each of the plurality of possible conversation topics for the second user, the knowledge base that stores associations between conversation topics and types of activities and/or behaviours, and one or more sensors for measuring activities and/or behaviours of the second user.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram of a system that includes an apparatus according to an aspect; and
Fig. 2 is a flow chart illustrating a method according to an embodiment.

### DETAILED DESCRIPTION OF EMBODIMENTS

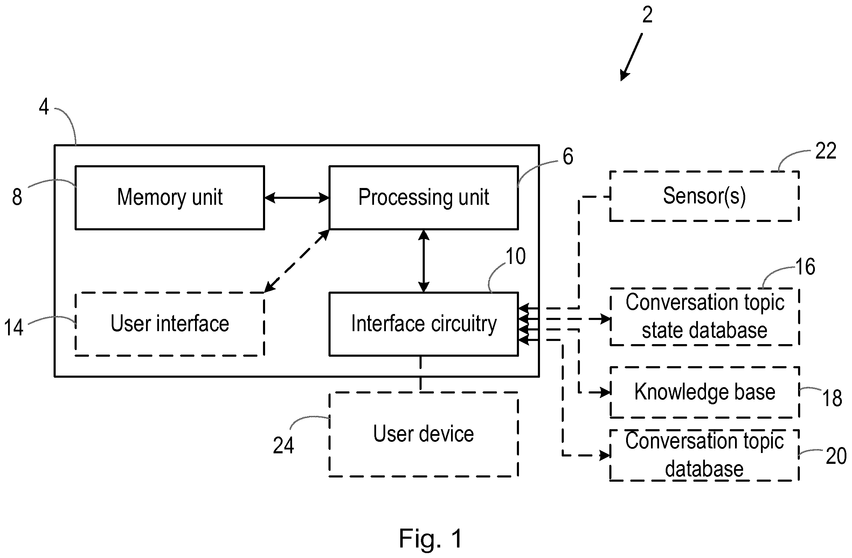

Fig. 1 is a block diagram of a system 2 that includes an apparatus 4 for providing information to a first user according to an aspect. The apparatus 4 includes a processing unit 6 that controls the operation of the apparatus 4 and that can be configured to execute or perform the methods described herein. The processing unit 6 can be implemented in numerous ways, with software and/or hardware, to perform the various functions described herein. The processing unit 6 may comprise one or more microprocessors or digital signal processor (DSPs) that may be programmed using software or computer program code to perform the required functions and/or to control components of the processing unit 6 to effect the required functions. The processing unit 6 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 6 is connected to a memory unit 8 that can store data, information and/or signals for use by the processing unit 6 in controlling the operation of the apparatus 4 and/or in executing or performing the methods described herein. In some implementations the memory unit 8 stores computer-readable code that can be executed by the processing unit 6 so that the processing unit 6 performs one or more functions, including the methods described herein. The memory unit 8 can comprise any type of non-transitory machine-readable medium, such as cache or system memory including volatile and nonvolatile computer memory such as random access memory (RAM) static RAM (SRAM), dynamic RAM (DRAM), read-only memory (ROM), programmable ROM (PROM), erasable PROM (EPROM), and electrically erasable PROM (EEPROM).

The apparatus 4 also includes interface circuitry 10 for enabling a data connection to and/or data exchange with other devices, including any one or more of servers, databases, user devices, and sensors. The interface circuitry 10 can enable a connection between the apparatus 4 and a network, such as the Internet, via any desirable wired or wireless communication protocol. For example, the interface circuitry 10 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). The interface circuitry 10 is connected to the processing unit 6.

In some embodiments, the apparatus 4 comprises a user interface 14 that includes one or more components that enables a user of apparatus 4 to input information, data and/or commands into the apparatus 4, and/or enables the apparatus 4 to output information or data to the user of the apparatus 4. The user interface 14 can comprise any suitable input component(s), including but not limited to a keyboard, keypad, one or more buttons, switches or dials, a mouse, a track pad, a touchscreen, a stylus, a camera, a microphone, etc., and the user interface 14 can comprise any suitable output component(s), including but not limited to a display screen, one or more lights or light elements, one or more loudspeakers, a vibrating element, etc.

The apparatus 4 can be any type of electronic device or computing device. For example the apparatus 4 can be, or be part of, a server, a computer, a laptop, a tablet, a smartphone, etc.

In various embodiments of the techniques described herein, the processing unit 6 requires information that can be used to identify conversation topics to suggest for a particular user. This information can be part of a database that is stored in memory unit 8, or it can be stored in a separate database 16 that is external to the apparatus 4. In that case, the processing unit 6 can retrieve information from the conversation topic state database 16 via the interface circuitry 10 as required. In either case, the processing unit 6 is able to interface with the database 16, i.e. to send information (e.g. queries and database content updates) to the database 16 and/or receive information (e.g. database content) from the database 16. This database 16 is referred to herein as a first database 16 or a conversation topic state database 16. The information that can be stored in the conversation topic state database 16 is described in more detail below.

In some embodiments, the system 2 can also comprise one or more additional databases that store other information that can be used as part of identifying conversation topics to suggest for a particular user. As with the conversation topic state database 16, this other information can be part of one or more additional databases that are stored in memory unit 8, or it or they can be stored in databases 18, 20 that are external to the apparatus 4. In that case, the processing unit 6 can interface with (e.g. retrieve information from) the one or more separate databases 18, 20 via the interface circuitry 10 as required. One such additional database can comprise a knowledge base 18 that stores information relating to associations between conversation topics and types of activities and/or behaviours of a user. Another such additional database can comprise a conversation topic database 20 (also referred to herein as a second database 20) that stores possible conversation topics for a particular user.

In various embodiments of the techniques described herein, the processing unit 6 requires information on the activities and/or behaviours of a person. This information can be obtained by one or more sensors 22 that monitor the person. The sensor(s) 22 may be part of the apparatus 4, or they may be separate from the apparatus 4. In the latter case, the sensor(s) 22 may be connected to the apparatus 4 via the interface circuitry 10. Alternatively, measurements by the sensor(s) 22 can be stored in a separate device or apparatus (not shown in Fig. 1), and the apparatus 4 can obtain the measurements from that separate device or apparatus. Various examples of the sensor(s) 22 are provided below.

As noted above, in some embodiments the apparatus 4 includes a user interface 14 that can include one or more output components for outputting information to a user of the apparatus 4/system 2 and/or one or more input components for enabling a user to input information into the apparatus 4/system 2. This output information can include one or more conversation topics that are determined according to the techniques described herein, and the information can be output in response to a signal or control signal from the processing unit 6. In alternative embodiments, for example where the apparatus 4 is a server or other electronic device that is not local to the user that requires the information on the conversation topics, the information on the conversation topics can be sent in the form of a signal from the apparatus 4 (processing unit 6) to a user device 24 that is being used by the person that requires the information (e.g. a care provider). The user device 24 can be any suitable type of device, including, but not limited to, a desktop computer, a laptop, a tablet, a smartphone, etc. The input information may be information indicating one or more possible conversation topics for the second user, information indicating the length of time and/or when a conversation topic was previously discussed, an indication that the first user requires conversation topics to be generated and presented to them, and/or any other information that is required or can be input by the first user for the operation of the apparatus 4/system 2 or performance of the techniques described herein.

It will be appreciated that a practical implementation of an apparatus 4 may include additional components to those shown in Fig. 1. For example the apparatus 4 may also include a power supply, such as a battery, or components for enabling the apparatus 4 to be connected to a mains power supply.

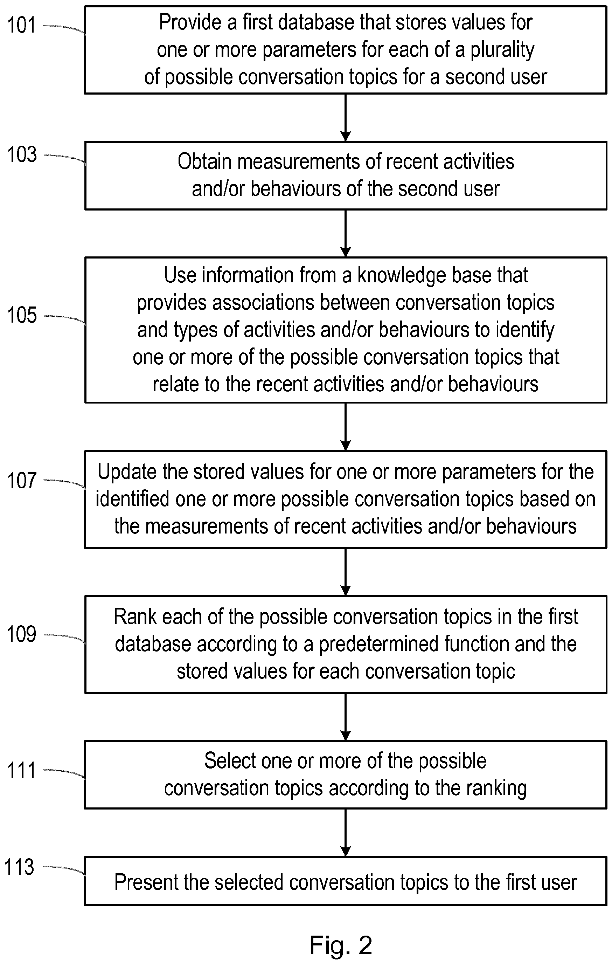

The flow chart in Fig. 2 illustrates a method of generating information for a first user, such as, in some embodiments, a family member or care provider, to improve their conversations with another person (who is referred to herein as a 'second user'). The information generated and output by the method to the first user includes one or more conversation topics that will enable the first user to initiate useful conversations with the second user. It will be appreciated that in alternative embodiments the first user can be an electronic conversation system (including a speech/voice technology), such as an artificial intelligence (AI) system or virtual assistant, that can operate to conduct a conversation with the second user. The method in Fig. 2 can be implemented by the apparatus 4, and in particular by the processing unit 6. As noted below, some or all steps of the method in Fig. 2 can be performed at any desired time, for example, periodically or prior to an expected interaction between the first user and the second user so that conversation topics are available to the first user when required, or the method can be performed whenever a new conversation topic is required by the first user (e.g. during a conversation with the second user).

Firstly, in step 101, the conversation topic state database 16 can be provided. This conversation topic state database 16 stores values for one or more parameters for each of a plurality of possible conversation topics for the second user. Thus, step 101 can comprise storing values for the one or more parameters for the plurality of possible conversation topics in the state database 16.

The values for the one or more parameters stored in the conversation topic state database 16 for each possible conversation topic are user-specific values for the second user. These one or more parameters can relate to the importance of the topic to the second user, an importance of the topic to the first user (or a family member of the second user), how recently the topic has been discussed or raised, how frequently the topic is discussed, and/or how long the topic was discussed for.

Thus, for each possible conversation topic (i.e. each row in conversation topic state database 16) one or more of the following can be stored:
- PI: an importance value/interest level indicating the level of importance or interest of the possible conversation topic to the second user or the first user;
- RC: a value indicating how recently the possible conversation topic has been raised or discussed (e.g. computed from the time of conversation, stored in next entry, and time of retrieval from the database 16 or conversation topic database 20);
- EG: a value indicating the time and duration of engagement with that possible conversation topic; and
- CV: a value indicating the time and frequency of when that possible conversation topic has been talked about before.

While the first two parameters above are typically stored once, i.e. appear in a single row in one table of the conversation topic state database 16, the last two parameters are typically stored multiple times, namely per engagement on the topic, and are typically stored as a second table of the conversation topic state database 16, i.e. a row per engagement. The rows in the second table associated with a same topic can be summarised in a single value which is stored in the first table. For example the average of all the durations as retrieved from the second database is stored is stored as 'duration' in the first table. This value can be dynamically updated.

In some embodiments, the one or more parameters stored in the conversation topic state database 16 for each possible conversation topic can also include one or more statistical values. These statistical values can be based on, or based partly on, the values of the exemplary one or more parameters outlined above, and/or other information such as information relating to recent behaviours and/or activities of the second user. For example the statistical value can be a mean value µ, a standard deviation value σ, and/or a median absolute deviation. These statistical values can be running values, and can be updated over time.

In some embodiments, the list of possible conversation topics for the second user is derived and stored separately from the conversation topic state database 16, and for example they are stored in conversation topic database (second database) 20. The conversation topic database 20 stores all possible conversation topics for the second user, and the conversation topic state database 16 stores values for the one or more parameters for each of the possible conversation topics.

The plurality of possible conversation topics may comprise any one or more of a default set of conversation topics, a default set of conversation topics for the second user, conversation topics relating to a health condition of the second user, conversation topics relating to regular activities of the user, conversation topics relating to current events, such as news events, entertainment (e.g. television, sport, etc.) events, the weather, etc., conversation topics specified by one or more family members of the second subject, conversation topics relating to responses by the second user to a questionnaire, etc. It will be appreciated that the list of possible conversation topics in the conversation topic database 20 is relatively static in the sense that it does not change significantly over short timescales (e.g. hours or days). This is in contrast to the content of the conversation topic state database 16 which can be updated more dynamically based on the activities and/or behaviours of the second user (as described below).

In step 103, measurements of recent activities and/or behaviours of the second user can be obtained. The measurements of the activities and/or behaviours of the second user can be obtained from memory unit 8 and/or they can be obtained directly from the one or more sensor(s). In either case, the measurements may be direct sensor measurements of the second user (e.g. the raw measurements from the sensor(s) 18), or the raw measurements may have been pre-processed to extract or identify one or more activities and/or behaviours from the raw sensor measurements.

As used herein, the activities and/or behaviours that can be measured include any activity or behaviour (including emotional state) that could provide useful or relevant information for determining a suitable or useful topic of conversation with the second user. The measurements of the activities and/or behaviour may include activities and/or behaviour during a particular period of time, for example the last hour, the last day, the time since the first user last spoke with the second user, etc. The activity or behaviour can include any of the following: the physical activities of the second user, a physical activity level, types of physical activity, location tracking of the second user (e.g. which rooms of a house has the second user been in, locations outside the house that the second user has visited), objects that the second user has interacted with (e.g. used a kettle, coffee maker, smartphone, computer etc.), the type of interaction with those objects (e.g. cooking food, posting on social media, viewing news items, television programs watched, streamed or downloaded, etc.), words spoken by the second user to the first user or another person, events that have taken place in the home of the second user, eating, drinking and/or sleeping patterns or habits of the second user, etc. Those skilled in the art will be aware of many other types of activities and/or behaviour that can be measured to provide inputs to the generation of conversation topics according to the techniques described herein, and thus the above list should not be considered limiting.

Any suitable types of sensors 18 can be used to measure these types of activities and behaviour (or provide measurements of the second user and/or their environment, from which measurements of the activities and/or behaviour can be derived). Depending on the type of sensor 18, the sensor 18 can be worn or carried by the second user, or the sensor 18 can be located in the environment around the second user, for example in the home environment of the second user. The one or more sensors 18 can include movement sensors for measuring the movements and/or locations of the second user, with the movement sensors including any of accelerometers, gyroscopes, magnetometers, satellite positioning system receivers, cameras, motion detectors such as passive infrared (PIR) sensors, door open/close sensors, pressure sensors, etc., electronic devices that the second user can interact with and that can monitor and output information on these interactions, such as televisions, smartphones, computers, kitchen appliances, etc., microphones (for listening to speech and conversations of the second user, etc.). It will be appreciated from the above that the one or more sensors 18 can include the so-called 'Internet of things', where household or everyday objects monitor their usage and are connected to the Internet to enable this information to be collected and analysed.

As noted above, the sensors 18 may output raw sensor measurements, and these measurements may be processed in order to determine or extract the activities and/or behaviours of the second user. This processing may be performed by the processing unit 6, or it may be performed by a separate device or apparatus. In either case, those skilled in the art will be aware of various algorithms and techniques for determining and/or extracting activities and/or behaviours from sensor measurements, and further details are not provided herein.

Next, in step 105, information from a knowledge base 18 is used to identify one or more of the possible conversation topics that relate to the recent activities and/or behaviours. As noted above, the knowledge base 18 can be stored in the memory unit 8 of the apparatus 4, or it can be stored separate from the apparatus 4. The knowledge base 18 provides associations between conversation topics and types of activities and/or behaviours, and for example stores one or more ontologies. Thus, the knowledge base 18 can store, for a plurality of conversation topics (e.g. tens, hundreds or thousands of possible topics), information that indicates a hierarchy for the topics, and information associating types of activities and/or behaviours with specific conversation topics. This stored information can, for example, enable the apparatus 4 to infer which topics are closely related to a found (health) state and (recent) daily activities of the (second) user.

The ontology or ontologies within the knowledge base 18 can be represented or expressed in the Web Ontology Language (OWL) which relates concepts to each other. Concepts are classes (i.e. a set of items), items (i.e. the instantiation of a class, which can be a class itself) and relationships (i.e. between classes and/or instances). Important (and usually predefined) relationships are "has" and "is a".

For example, [the instance] 'Jack' "is a" [member of the class] 'Human'. Further, 'Jack' "isParentOf" 'Joan'. In the ontology (knowledge base) it is defined (a rule that has been stored in the ontology earlier) that the inverse of "isParentOf" is "isChildOf". So, given the above three relationships, the ontology enables it to be inferred that 'Joan' "isChildOf" 'Jack'. Likewise, it can be inferred that 'Joan' "is a" 'Human'. This mechanism can be extended, so that, for example, diseases and daily activities are described and terms (words/topics) are related. As an example, when given 'toilet during night' as an activity, a link (path) with 'bad sleep' can be found using the ontology.

In the context of the techniques described herein, the ontology could include high level classes of topics, such as 'Social', 'Activities', 'Entertainment', 'Health' and 'Hygiene', with the next level of classes being 'visits', 'telephone calls', 'going out' for Social, 'entertainment', 'health' and 'hygiene' for Activities, 'TV watching', 'painting', 'gardening', 'walking in forest', 'hobbies' for Entertainment, 'sleeping', 'eating' for 'Health' and 'personal care', 'toileting' for Hygiene. The ontology enables individual topics to be evaluated with consideration given to observed (recent) activities by the user and to the health state of the user.

The knowledge base can consist of several ontologies, for example as described in "An overview of ontologies and data resources in medical domains" by Mirjana Ivanovic, Expert Systems with Applications, vol. 41, issue 11, 1 September 2014, pages 5158-5166. In addition or as an alternative to these ontologies, other ontologies can be used that have been constructed specifically for the system 2/apparatus 4, for example that include concepts such as topics, (daily) activities, health, and their relations.

Next, in step 107, the values for one or more of the parameters stored in the conversation topic state database 16 for the one or more possible conversation topics that relate to the recent activities and/or behaviours are updated based on the measurements of the recent activities and/or behaviours.

In some embodiments, in addition to the values of the one or more parameters, the conversation topic state database 16 also stores, for one or more of the plurality of possible conversation topics, information on recent and previous activities and/or behaviours of the second user relating to the conversation topic (or in some embodiments information on abnormal recent and previous activities and/or behaviours of the second user relating to the conversation topic). This information is derived from the measurements obtained in step 103 and is stored with associated conversation topics (as indicated by the information in the knowledge base 18.

Next, in step 109, when information (i.e. conversation topics to use) is to be generated for the first user, the method comprises ranking each of the possible conversation topics in the conversation topic state database 16 according to a predetermined function and the stored values for each possible conversation topic.

In some embodiments, step 109 can comprise using the predetermined function and the stored values for each possible conversation topic to determine a ranking value for each possible conversation topic. In some embodiments, the predetermined function takes the stored values for the one or more parameters as inputs to determine the ranking value.

In some embodiments, the predetermined function is a weighted sum of the input values for the one or more parameters. In some embodiments, cross dependencies can also be included. A machine learning algorithm can be used to adapt the weightings of the input parameters. This adaptation can be based on feedback by the first user, which feedback assigns a relevance/interest value to certain topics (e.g. "don't suggest this topic again", "give topics related to gardening"), etc.

In some embodiments, the predetermined function determines a normality value for each possible conversation topic for which an associated recent activity and/or behaviour has been measured. The normality value provides a measure of how normal or abnormal the associated recent activity and/or behaviour is for the second user. This normality value can be used in determining the ranking value. Determining the normality value can comprise comparing the activities and/or behaviours for the second user to a set of regular activities or behaviours for the second user, or to a set of regular activities or behaviours for a population of users (e.g. that have the same medical condition as the second user). In some embodiments, the normality value can be calculated as (x - g) / σ, where x is the number of occurrences of the activity and/or behaviour in a recent time period (e.g. a day), µ is the mean number of occurrences of the activity and/or behaviour over a longer time period (e.g. the preceding week or month), and σ is the standard deviation of the number of occurrences of the activity and/or behaviour over the longer time period (e.g. the preceding week or month). It may be that activities and/or behaviours that are unusual for the second subject may be useful conversation topics for the first user, and thus a normality value indicating an abnormal or unusual activity and/or behaviour can contribute to a higher ranking value for the associated conversation topic(s). In some embodiments, the normality value can be considered as another parameter alongside the PI, RC, EG and CV parameters outlined above, with a normality value being stored in the conversation topic state database 16 for particular behaviours and/or activities.

In some embodiments, step 109 can further comprise, for each possible conversation topic, searching a first external information source for information relevant to the possible conversation topic, and determining a weighting value for each conversation topic based on information relevant to the conversation topic that is found in the search of the first external information source. This weighting value is also referred to as an actuality value. The search can be for information on local or global events, e.g. recent news items or other items (e.g. website posts) that relate to the topic. Thus, the first external information source can be the Internet, for example news websites, weather websites, health websites, entertainment websites, etc. If any such news items or other items are found, a weighting (actuality) value can be assigned. This actuality value is a weighting and can be based on, for example, how recently the news items or other items were posted, the number of relevant Internet 'hits' for the topic, how high in the search results the news items or other items were ranked. In some embodiments, the weighting (actuality) value is used in the predetermined function to determine the ranking value. In other embodiments, the weighting (actuality) value can be applied to the output of the predetermined function (e.g. an initial ranking value) to determine a final ranking value.

As an example, in embodiments where an actuality value is determined, the predetermined function can act to prioritise (i.e. give a high ranking to) conversation topics that have a high actuality value e.g. topics that had a high value following the Internet search, and to provide a low ranking to or exclude those topics for which the weight is low (for example a health and hygiene class in the ontology) and/or to those topics for which the PI level is low and the normality value does not exceed a threshold (i.e. there is low interest in this topic for conversation).

Once the possible conversation topics have been ranked, one or more of the possible conversation topics are selected according to the ranking (step 111). Step 111 can comprise selecting a predetermined number of the highest ranking conversation topics. For example, the five or ten highest ranking conversation topics can be selected in step 111. Alternatively, step 111 can comprise comparing the determined ranking value to a threshold value and selecting all or a subset of the possible conversation topics for which the determined ranking value exceeds the threshold value. In some embodiments, the same threshold value can be used for all of the possible conversation topics. In other embodiments, each conversation topic can have a respective threshold value. This embodiment enables the threshold to act as a weighting factor that determines whether that particular topic is to be suggested.

Once one or more of the possible conversation topics have been selected, in step 113 the selected conversation topics are presented to the first user, i.e. the selected conversation topics are output to be presented to the first user. The selected conversation topics can be presented in any desired way. For example, the topics can be presented as a word cloud, e.g. based on key words or trigger words for the selected topics, the information can be presented as one or more questions that the first user can ask, or the information can be presented as one or more factual statements about the second user (e.g. the second user has not eaten today) so that the first user can formulate their own way to structure the conversation.

Where the first user directly uses the apparatus 4, the user interface 14 can be used to present the information to the first user (e.g. via a display screen in the user interface 14). Alternatively, where the first user has their own user device 20 (e.g. a smartphone), step 113 can comprise providing the information from the apparatus 4 to the user device 24 for display (or other type of presentation) to the first user. In this case, the smartphone can include an app for presenting this information to the first user. In some embodiments, the information from the apparatus 4 can be presented to both the first user and the second user, for example on a display screen, such as a television or computer monitor, in the environment around the first user and second user.

As an example of the above techniques, if the measurements of the recent activities and/or behaviours indicate that there is an increased number of toilet visits over the day and/or during the night, step 105 can link this behaviour to a conversation topic relating to urinary tract infections (UTIs), and thus the importance parameter (PI) of the UTI-related conversation topic can be increased in step 107. EG, or a comparable parameter, can also be increased when this increased number of visits occurs every night since last week, for example.

As another example relating to gardening, suppose the second user is known (by the first user) to like gardening. So, the measurements of the recent activities and/or behaviours in step 103 can indicate when and how long the second user spent in the garden. Changes in this behaviour may affect the EG parameter for conversation topics relating to gardening in step 107. The time of year and weather conditions (e.g. sourced via an Internet search) may serve as weighting factors in computing this EG in step 107, although they can be stored as separate items, and/or used in a subsequent step of the method (e.g. the ranking step 109). In this example, the ontology used in step 105 may relate 'gardening' behaviour to conversation topics like 'tulips', 'sowing', 'harvesting', 'mowing', which can be suggested to the first user for conversation: e.g. in spring and sunshine, if the measurements of activities and/or behaviours indicates that the second user has spent little time in the garden, the apparatus 4 can suggest to the first user to talk to the second user about sowing bulbs (e.g. crocus, tulips, etc.).

In further embodiments, the conversation topic database 20 can be updated periodically or frequently to include new conversation topics that are potentially relevant to the second user. Thus, a second external information source (such as the Internet) can be searched for external information relevant to the second user. The conversation topic database 20 is updated with one or more new conversation topics that relate to the external information (e.g. a news event), and then the conversation topic state database 16 is updated to store values for the one or more parameters for each of the one or more new conversation topics. The conversation topic database 20 (and therefore the conversation topic state database 16) can likewise be updated to include new conversation topics in response to inputs by the first user, the second user and/or any other relevant party (e.g. a family member).

In some embodiments, a user profile may be provided for the second user that indicates importance values to the second user of one or more of the plurality of possible conversation topics stored in the conversation topic database 20. The user profile may be provided by the first user, the second user and/or any other relevant party (e.g. a family member). The user profile information may be used to set or update the values of one or more of the parameters stored in the conversation topic state database 16. In some embodiments, the importance value (PI) for each conversation topic in the conversation topic state database 16 can be determined based on a user profile for the second user that indicates preferences, interests, friends, hobbies, etc. for the second user.

In some embodiments, after presenting the selected conversation topics to the first user, the values for the one or more parameters for the selected conversation topics stored in the conversation topic state database 16 can be updated. For example, the values of the parameters relating to the interest level of the second user in the topic, how recently the topic has been discussed, the time and duration of engagement with that topic and/or the time and frequency of when that conversation topic has been talked about before can be updated.

In a particular embodiment, feedback can be provided by the first user and/or the second user on a selected conversation topic (for example after the conversation topic has been discussed by the first user and the second user), and this feedback can be used to update the stored values for the one or more parameters for the selected conversation topics. In particular, the feedback can be used to update any one or more of the PI, RC, time and duration of engagement, and time and frequency of when that conversation topic was talked about before.

In some embodiments, the feedback provided by the first user and/or the second user can be factored into the predetermined function to apply feedback or scoring from the first user based on previous conversations with the second user in determining a ranking for the possible conversation topics. For example, if a topic was previously suggested to the first user as a conversation topic with the second user, the first user may have provided feedback that the second user did/did not engage with the topic, or that it did/did not result in a useful conversation.

A specific example of the techniques described above is provided for the generating conversation topics within the 'entertainment' class of conversation topics. For the topic 'gardening' (e.g. which can be detected based on the time that the second user spent outside the house after leaving by the back door), the importance value (PI) can be, e.g., 0.7 (which indicates a high importance to the second user, with a scale of values from 0 to 1), and the RC value (how recent the second user did this activity) might be 72 hours. Step 103 can comprise detecting a gardening activity through the use of the back door without the second user returning to the house for more than 10 minutes during the day. The time and duration of the detected gardening activity can be added to the gardening entries in the conversation topic state database 16. Next, in step 109, the normality value is calculated, expressed by mean µ and standard deviation σ. For example, mean time is 10:15 AM with standard deviation of 30 minutes. When recent activity lists 16:45, this may be taken as unusual, although, perhaps not worrying, if the duration is corresponding with the mean for that parameter. The abnormality is a good topic to suggest: to chat about gardening (the second user likes such and might have enjoyed some more that afternoon). It will be appreciated that normality is modelled as a random distribution. The actuality value is calculated by searching for relevant news on the Internet, including weather events. The search reveals news items concerning heavy rain, so the actuality value is also high. Thus, in step 109, the gardening topic is ranked against all other possible topics using the predetermined ranking function. This function can calculate a rank score based on normality, actuality, PI, RC, EG and/or CV, and in this case the gardening topic ranks highly. Therefore this topic is selected in step 111 to be presented to the first user as a suggested topic of conversation. This might mean including words relating to gardening in a word cloud, and/or providing relevant questions such as "Did you get in before the rain?" or "How is the garden today?"

As a further example, the apparatus 4 can infer that a visitor was recently in the second user's house through measurements from presence sensors (e.g. PIR sensors), with the second user being identified using an RF token for a burglar alarm. This information can lead to a conversation topic about a recent visitor being suggested by the apparatus 4.

In another example, the apparatus 4 can identify that the second user watched a particular TV programme through monitoring a television or computer, or using a camera and/or microphone in the environment, and this information can lead to a conversation topic about recent TV habits or the specific TV programme being suggested.

In another example the apparatus 4 can identify that the second user repetitively contacted a close family member (e.g. via telephone) an abnormal amount of times. For example, the second user is worried about something, gets anxious and repetitively calls another family member about that subject.

Thus, the techniques set out above provide that the system 2/apparatus 4 identifies conversation topics by processing data from one or more sensors 18, for example arranged in a 'smart home' arrangement, to determine which activities have been taking place, and a list of these topics can be presented to the first user. This list is therefore an 'informed' list of conversation topics and is much more likely to be useful for creating an engaging and flowing conversation than a generic one.

There is therefore provided a method and apparatus for providing or suggestion conversation topics to a user to enable them to improve their conversations with another user.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method to generate information for a first user, the method comprising:
storing, via a processing unit, values for one or more parameters for each of a plurality of possible conversation topics for a second user in a first database;
obtaining, via the processing unit, measurements of recent activities and/or behaviours of the second user;
using, via the processing unit, information from a knowledge base that provides associations between conversation topics and types of activities and/or behaviours to identifyone or more of the possible conversation topics that relate to the recent activities and/or behaviours;
updating, via the processing unit, the stored values for one or more parameters for the identified one or more possible conversation topics based on the measurements of recent activities and/or behaviours; and
when information is to be generated for the first user:
ranking, via the processing unit, each of the possible conversation topics according to a predetermined function and the stored values for each possible conversation topic;
selecting, via the processing unit, one or more of the possible conversation topics according to the ranking; and
outputting the selected conversation topics to be presented to the first user.

2. A computer-implemented method as claimed in claim 1, wherein the one or more parameters stored in the first database for each of the plurality of possible conversation topics for the second user relate to one or more of:
- an importance of the conversation topic to the first user and/or the second user;
- how recently the conversation topic was used for the second user;
- a duration of a previous conversation with the second user on the conversation topic; and
- how frequently the conversation topic is used for the second user.

3. A computer-implemented method as claimed in claim 1 or 2, wherein the predetermined function comprises determining, for each possible conversation topic for which an associated recent activity and/or behaviour has been measured, a normality value that provides a measure of how normal or abnormal the associated recent activity and/or behaviour is for the second user.

4. A computer-implemented method as claimed in any of claims 1-3, wherein the step of ranking each of the possible conversation topics comprises, for each conversation topic in the first database, searching an external information source for information relevant to the conversation topic;
wherein the predetermined function comprises determining a weighting value for each conversation topic based on information relevant to the conversation topic that is found in the search of the external information source.

5. A computer-implemented method as claimed in any of claims 1-4, wherein the method further comprises:
evaluating, via the processing unit, the obtained measurements of the recent activities and/or behaviours of the second user to determine whether any of the recent activities and/or behaviours are abnormal for the second user; and
storing, via the processing unit, for one or more of the plurality of possible conversation topics, information on determined abnormal recent activities and/or behaviours of the second user relating to the conversation topic.

6. A computer-implemented method as claimed in any of claims 1-5, wherein the method further comprises:
searching, via the processor, an external information source for external information relevant to the second user;
updating, via the processing unit, a second database that stores the plurality of possible conversation topics for the second user with one or more new conversation topics relating to the external information; and
updating, via the processing unit, the first database to store values for the one or more parameters for each of the one or more new conversation topics.

7. A computer-implemented method as claimed in any of claims 1-6, wherein the method further comprises:
obtaining, via the processing unit, user profile information relating to the second user, the user profile information indicating importance values for one or more of the plurality of possible conversation topics for the second user; and
updating, via the processing unit, the stored values for the one or more parameters for the one or more of the plurality of possible conversation topics for the second user based on the user profile information.

8. A computer-implemented method as claimed in any of claims 1-7, wherein the method further comprises:
after presenting the selected conversation topics to the first user, updating, via the processing unit, the stored values for the one or more parameters for the selected conversation topics.

9. A computer-implemented method as claimed in any of claims 1-8, wherein the method further comprises:
receiving, via the processing unit, feedback from the first user and/or the second user on the selected conversation topics; and
updating, via the processing unit, the stored values for the one or more parameters for the selected conversation topics based on the received feedback.

10. A computer program product comprising a computer readable medium having computer readable code embodied therein, the computer readable code being configured such that, on execution by a suitable computer or processor, the computer or processor is caused to perform the method of any of claims 1-9.

11. An apparatus for generating information for a first user, the apparatus comprising:
a processing unit that is configured to:
interface with a first database that stores values for one or more parameters for each of a plurality of possible conversation topics for a second user;
obtain measurements of recent activities and/or behaviours of the second user;
use information from a knowledge base that provides associations between conversation topics and types of activities and/or behaviours to identify one or more of the possible conversation topics that relate to the recent activities and/or behaviours;
update the stored values for one or more parameters for the identified one or more possible conversation topics based on the measurements of recent activities and/or behaviours;
rank each of the possible conversation topics in the first database according to a predetermined function and the stored values for each possible conversation topic when information is to be generated for the first user;
select one or more of the possible conversation topics according to the ranking; and
provide a signal to a user interface to present the selected conversation topics to the first user.

12. An apparatus as claimed in claim 11, wherein the processing unit is further configured to:
evaluate the obtained measurements of the recent activities and/or behaviours of the second user to determine whether any of the recent activities and/or behaviours are abnormal for the second user; and
store, in the first database, for one or more of the plurality of possible conversation topics, information on determined abnormal recent activities and/or behaviours of the second user relating to the conversation topic.

13. An apparatus as claimed in any of claims 11 or 12, wherein the processing unit is further configured to:
obtain user profile information relating to the second user, the user profile information indicating importance values for one or more of the plurality of possible conversation topics for the second user; and
update the stored values for the one or more parameters for the one or more of the plurality of possible conversation topics for the second user based on the user profile information.

14. An apparatus as claimed in any of claims 11-13, wherein the processing unit is further configured to:
receive feedback from the first user and/or the second user on the selected conversation topics; and
update the stored values for the one or more parameters for the selected conversation topics based on the received feedback.

15. A system comprising:
an apparatus according to any of claims 11-14; and
one or more of:
the first database that is configured to store values for one or more parameters for each of the plurality of possible conversation topics for the second user,
the knowledge base that stores associations between conversation topics and types of activities and/or behaviours, and
one or more sensors for measuring activities and/or behaviours of the second user.
